# EUROPEAN PATENT SPECIFICATION

(11) **EP 2 286 675 B1**
(45) Date of publication and mention of the grant of the patent: **23.05.2012**
(21) Application number: 10176057.7
(22) Date of filing: 10.12.2002
(51) Int. Cl.: A23L 1/30, A61K 36/23, A61P 19/00

(54) **Composition for promotion of bone growth and maintenance of bone health**
Zusammensetzung für die Förderung des Knochenwachstums und die Erhaltung der Knochengesundheit
Composition pour la promotion de la croissance osseuse et la conservation de la santé osseuse

(30) Priority: 11.12.2001 EP 01204838
(43) Date of publication of application: 23.02.2011
(62) Divisional of application: 09172445.0
(73) Proprietor: Société des Produits Nestlé S.A., 1800 Vevey (CH)
(72) Inventor: Offord-Cavin, Elizabeth, CH-1820 Montreux (CH); Federici, Ermanno, Oxford, OX3 7NP (GB); Lemaure, Bernard, 37000 Tours (FR); Courtois, Didier, F37550 St Avertin (FR)
(74) Representative: Brearley, Helen Rebecca

(56) References cited:
- WO-A-00/64438
- WO-A-01/01996
- WO-A-01/03716
- WO-A-01/45725
- WO-A-99/47149
- WO-A-99/55351
- CN-A- 1 169 863
- DE-A- 19 814 725
- GB-A- 2 355 189
- US-A- 5 908 628
- US-A1- 2001 012 523
- US-A1- 2001 024 664
- US-B1- 6 235 287
- US-B1- 6 274 176
- DATABASE WPI Section Ch, Week 200204 Thomson Scientific, London, GB; Class B04, AN 2002-017683 XP002198191, -& JP 2001 302539 A (MEIJI SEIKA KAISHA LTD) 31 October 2001 (2001-10-31)
- DATABASE WPI Section Ch, Week 200163 Thomson Scientific, London, GB; Class B04, AN 2001-558222 XP002198192, & CN 1 302 636 A (TAN C) 11 July 2001 (2001-07-11)
- DATABASE WPI Section Ch, Week 200219 Thomson Scientific, London, GB; Class B04, AN 2002-140742 XP002198193, & CN 1 322 553 A (CHEN Z) 21 November 2001 (2001-11-21)
- DATABASE WPI Section Ch, Week 200176 Thomson Scientific, London, GB; Class B04, AN 2001-657430 XP002198194, & CN 1 307 887 A (KONG Z) 15 August 2001 (2001-08-15)
- DATABASE WPI Section Ch, Week 200144 Thomson Scientific, London, GB; Class B04, AN 2001-409765 XP002198195, & CN 1 292 274 A (CHEN Z) 25 April 2001 (2001-04-25)
- DATABASE WPI Section Ch, Week 200120 Thomson Scientific, London, GB; Class B04, AN 2001-192235 XP002198196, & CN 1 274 599 A (ZHOU J) 29 November 2000 (2000-11-29)
- DATABASE WPI Section Ch, Week 200127 Thomson Scientific, London, GB; Class B04, AN 2001-264633 XP002198197, & RU 2 162 334 C (TITIEVA N M) 27 January 2001 (2001-01-27)
- DATABASE WPI Section Ch, Week 200103 Thomson Scientific, London, GB; Class B04, AN 2001-016794 XP002198198, & CN 1 267 526 A (CHEN Y) 27 September 2000 (2000-09-27)
- DATABASE WPI Section Ch, Week 199836 Thomson Scientific, London, GB; Class D16, AN 1998-426120 XP002198409, & RU 2 102 460 C (UFA WINE WKS) 20 January 1998 (1998-01-20)

## Description

The present invention relates to a composition for maintenance of bone health or prevention, alleviation and/or treatment of bone disorders.

### Background of the Invention

Healthy bones require effective bone remodelling involving an equilibrium between bone formation and resorption. Most bone diseases are due to increased bone resorption, rendering its inhibition a primary therapeutic objective, therefore most pharmaceutical agents, developed to date, are anti-resorptive. For example, estrogens block production of cytokines that promote osteoclast generation and differentiation. SERM_{S} (selective estrogen response modulator) are being developed which provide benefits for bone health while reducing the risk of adverse hormonal effects on breast or endometrial tissues. It is assumed that they work by a similar mechanism to estrogen in bone. Bisphosphonates (such as alendronate, risedronate etc) concentrate in bone and are, to date, the most effective inhibitors of bone resorption. They inhibit a critical enzyme pathway, required for osteoclast activity and survival. Calcitonin is a polypeptide hormone that inhibits bone resorption by blocking osteoclast activity. New targets include blocking of the TNF receptor/ligand family members and their signalling pathways, particularly of RANK/RANKL, inhibition of bone-specific metalloproteinases such as cathepsin K or inhibition of specific kinases.

Development of therapeutic agents stimulating bone formation has lagged behind that of resorption. Some chemical or pharmaceutical agents are known for promoting bone growth in humans. For example, WO 9619246 describes a method for promoting bone growth in a human patient by intermittent administration of parathyroid hormone, PTH-related protein or an agonist for at least one month. In WO 9619501, a pancreatic-derived factor inhibits the resorption of bone and stimulates bone cells to proliferate and increases the formation of bone.

A major recent breakthrough has been the demonstration of the role of bone morphogenic protein 2 (BMP-2) as a key player in stimulation of bone formation and that statins (effective drugs for cholesterol-lowering through inhibition of cholesterol synthesis) improve bone formation, partly mediated by induction of BMP-2. The delivery of recombinant BMP-2 has been shown to induce bone or cartilage formation. In US 6150328, a method for the induction of bone and cartilage formation comprises administering a purified bone morphogenic protein produced by culturing a cell transformed with DNA encoding BMP, is described. Also, WO 9711095 relates to the use of bone morphogenic protein compositions for treatment of neural tumours and bone growth and wound healing. In addition to BMPs, growth factors such as insulin-like GF (IGF-1), transforming GF-β (TGF-β), fibroblast GFs (FGFs) are under investigation as local therapies in the healing of fractures and bone defects. However, systemic administration is problematic due to the metabolism in the intestine and also due to possible effects on other tissues. Gene therapy is being proposed as one option. The alternative is to target osteoblast regulation of their production by dietary or pharmaceutical regulators of their gene or protein expression.

Although these chemicals and pharmaceutical compounds have been proved for the treatment of bone disorders, it would be of interest to provide a safe, efficient nutritional way to promote bone growth and prevent, alleviate or treat bone/joint disorders in mammals.

US 2001/0024664 relates to a method for inhibiting COX-2 in an organism by administering an organic extract isolated from an edible plant.

### Summary of the Invention

Accordingly, in a first aspect, the present invention provides a composition for the prevention, alleviation and/or treatment of bone disorders or maintenance of bone health in mammals, which comprises as an active ingredient an effective amount of at least one plant or plant extract selected for its ability to induce bone morphogenic protein expression, wherein the plant or plant extract is *Carum.*

Remarkably, it has now been found that *Carum* plants or plant extracts have the ability to promote bone growth through regulation of endogenous growth factors in bone or cartilage tissue. They have the ability to induce bone morphogenic protein expression in vivo and on local bone environment, which has a positive effect on bone formation and repair, on maintenance of bone health or prevention, alleviation and/or treatment of bone disorders.

The composition according to the invention can be used in the manufacture of a nutritional product, a supplement, a treat or a medicament intended for humans or pets.

Administering to an individual a food composition according to the present invention, results in an improved bone regeneration during fracture healing. It helps to increase bone formation and bone mineral density during growth and optimize peak bone mass. It helps to maintain bone mass with age and reduces the risk of osteoporosis.

Furthermore it helps to build cartilage in mammals, to prevent osteoarthritis in pets or humans, which results in a better activity or mobility of the individual.

In another aspect, the invention relates to the use of a plant or a plant extract selected for their ability to stimulate bone morphogenic proteins, for the preparation of a composition for use in;
helping bone regeneration during fracture healing;
helping to increase bone formation and bone mineral density during growth;
optimizing peak bone mass;
helping to build cartilage;
helping to prevent osteoarthritis which results in a better activity or mobility of the individual;
the treatment, alleviation and/or prophylaxis of osteoarthritis;
treating or preventing osteoporosis;
in humans or pets.

The plant or plant extract is *Carum.*

### Detailed Description of the Invention

With respect to the first object of the present invention, the *Carum* plant or plant extract according to the invention contains phytochemicals having an anabolic potential through induction of bone morphogenic protein expression.

In a preferred embodiment, the plant or plant extract is from any part of the plant source, e. g. leaves, tubers, fruits, seeds, roots, grains, embryos or cell cultures. The plant or plant extract may be in the form of a dried, lyophilized extract of leaves, roots and/or fruits depending on the source of plant, or fresh plant, or enriched fraction obtained by inorganic or organic solvent extraction process known in the art.

In a most preferred embodiment it may be *Carum carvi.*

The phytochemicals may be carvone.

The plant or plant extract according to the invention may be used in the preparation of a food composition. The said composition may be in the form of a nutritionally balanced food or pet food, a dietary supplement, a treat or a pharmaceutical composition.

The plant or plant extract may be used alone or in association with other plants such as chicory, tea, cocoa, or with other bioactive molecules such as antioxidants, fatty acids, prebiotic fibres, glucosamine, chondroitin sulphate, for example.

In one embodiment, a food composition for human consumption is prepared. This composition may be a nutritional complete formula, a dairy product, a chilled or shelf stable beverage, soup, a dietary supplement, a meal replacement, and a nutritional bar or a confectionery.

Apart from the plant extract according to the invention, the nutritional formula may comprise a source of protein. Dietary proteins are preferably used as a source of protein. The dietary proteins may be any suitable dietary protein; for example animal proteins (such as milk proteins, meat proteins and egg proteins); vegetable proteins (such as soy protein, wheat protein, rice protein, and pea protein); mixtures of free amino acids; or combinations thereof. Milk proteins such as casein, whey proteins and soy proteins are particularly preferred. The composition may also contain a source of carbohydrates and a source of fat.

If the nutritional formula includes a fat source, the fat source preferably provides about 5% to about 55% of the energy of the nutritional formula; for example about 20% to about 50% of the energy. The lipids making up the fat source may be any suitable fat or fat mixtures. Vegetable fats are particularly suitable; for example soy oil, palm oil, coconut oil, safflower oil, sunflower oil, corn oil, canola oil, lecithins, and the like. Animal fats such as milk fats may also be added if desired.

A source of carbohydrate may be added to the nutritional formula. It preferably provides about 40% to about 80% of the energy of the nutritional composition. Any suitable carbohydrates may be used, for example sucrose, lactose, glucose, fructose, corn syrup solids, and maltodextrins, and mixtures thereof. Dietary fibre may also be added if desired. If used, it preferably comprises up to about 5% of the energy of the nutritional formula. The dietary fibre may be from any suitable origin, including for example soy, pea, oat, pectin, guar gum, gum arabic, and fructooligosaccharides. Suitable vitamins and minerals may be included in the nutritional formula in an amount to meet the appropriate guidelines.

One or more food grade emulsifiers may be incorporated into the nutritional formula if desired; for example diacetyl tartaric acid esters of mono- and di- glycerides, lecithin and mono- and di-glycerides. Similarly suitable salts and stabilisers may be included. Vitamins and minerals may also be combined with the plant extract.

The nutritional composition is preferably enterally administrable; for example in the form of a powder, tablet, capsule, a liquid concentrate, solid product or a ready-to-drink beverage. If it is desired to produce a powdered nutritional formula, the homogenised mixture is transferred to a suitable drying apparatus such as a spray drier or freeze drier and converted to powder.

In another embodiment, a nutritional composition comprises a milk-based cereal together with a prebiotic formulation. Preferably the milk-based cereal is an infant cereal which acts as a carrier for the prebiotic formulation.

In another embodiment, a usual food product may be enriched with at least one plant or plant extract according to the present invention. For example, a fermented milk, a yoghurt, a fresh cheese, a renneted milk, article of confectionery, for example a sweet or sweetened beverage, a confectionery bar, breakfast cereal flakes or bars, drinks, milk powders, soy-based products, non-milk fermented products or nutritional supplements for clinical nutrition.

The amount of the plant or plant extract in the composition may vary according to the plant source and its utilization. In a preferred embodiment, an efficient daily dose amount is of at least about 1 mg, and more preferably from 1 mg to 200mg of the active molecule per day.

In one embodiment, a pharmaceutical composition containing at least an extract or phytochemical as described above, in an amount sufficient to achieve the desired effect in an individual can be prepared. This composition may be a tablet, a liquid, capsules, soft capsules, pastes or pastilles, gums, or drinkable solutions or emulsions a dried oral supplement, a wet oral supplement. The pharmaceutical composition will further contain carriers and excipients that are suitable for delivering the respective active molecule of different nature to the target tissue. The kind of the carrier/excipient and the amount thereof will depend on the nature of the substance and the mode of drug delivery and/or administration contemplated. It will be appreciated that the skilled person will, based on his own knowledge select the appropriate components and galenic form.

The plant or plant extract according to the invention may be used in the preparation of a pet food composition. The said composition may be administered to the pet as a supplement to its normal diet or as a component of a nutritionally complete pet food, and more preferably in an hypocaloric pet food. It may also be a pharmaceutical composition.

The plant or plant extract may be used alone or in association with other plants such as chicory, tea, cocoa, or with other bioactive molecule such as antioxidants, fatty acids, prebiotic fibers, glucosamine, chondroitin sulphate for example.

Preferably, the pet food composition contains about 0.01 to 0.5 g of dry plants per gram of dry pet food for a 15 kg dog; and 0.001 to 0.1 g of dry plants per gram of wet pet food for a 15 kg dog.

The nutritionally complete pet food composition according to the invention may be in powdered, dried form, a treat or a wet, chilled or shelf stable pet food product. It may be chilled or provided as a shelf stable product. These pet foods may be produced by ways known in the art.

The pet food may optionally also contain a prebiotic, a probiotic microorganism or another active agent, for example a long chain fatty acid. The amount of prebiotic in the pet food is preferably less than 10% by weight. For example, the prebiotic may comprise about 0.1% to about 5% by weight of the pet food. For pet foods which use chicory as the source of the prebiotic, the chicory may be included to comprise about 0.5% to about 10% by weight of the feed mixture; more preferably about 1% to about 5% by weight

If a probiotic micro-organism is used, the pet food preferably contains about 10⁴ to about 10¹⁰ cells of the probiotic micro-organism per gram of the pet food; more preferably about 10⁶ to about 10⁸ cells of the probiotic micro-organism per gram. The pet food may contain about 0.5% to about 20% by weight of the mixture of the probiotic micro-organism; preferably about 1% to about 6% by weight; for example about 3% to about 6% by weight.

If necessary, the pet food is supplemented with minerals and vitamins so that they are nutritionally complete. Further, various other ingredients, for example, sugar, salt, spices, seasonings, flavouring agents, and the like may also be incorporated into the pet food as desired.

In another embodiment, dietary adjuncts may be prepared so as to improve pet food quality. As dietary adjuncts, they may be encapsulated or may be provided in powder form and packaged in conjunction with or separately from a main meal, be it wet or dry. By way of example, a powder containing extracts according to the invention, may be packed in sachets in a powder form or in a gel or lipid or other suitable carrier. These separately packaged units may be provided together with a main meal or in multi-unit packs for use with a main meal or treat, according to user instructions.

The amount of pet food to be consumed by the pet to obtain a beneficial effect will depend on the size of the pet, the type of pet, and age of the pet. However, an amount of the pet food to provide a daily amount of about 0.5 to 5 g of dry plants per kg of body weight, would usually be adequate for dogs and cats.

Administering to a human or animal, the food or pet food composition as described above, results in an improved bone regeneration during fracture healing. It helps to stimulate bone formation and bone mineral density during growth and optimize peak bone mass. In particular it may provide an optimal bone growth during childhood.

It reduces risk of osteoporosis and improves recovery after fracture.
Furthermore it helps to build cartilage in mammals, prevent osteoarthritis in pets and humans, which results in a better activity or mobility of the individual.

The following examples are given by way of illustration only and in no way should be construed as limiting the subject matter of the present application. All percentages are given by weight unless otherwise indicated. The examples are preceded by a brief description of the figure.

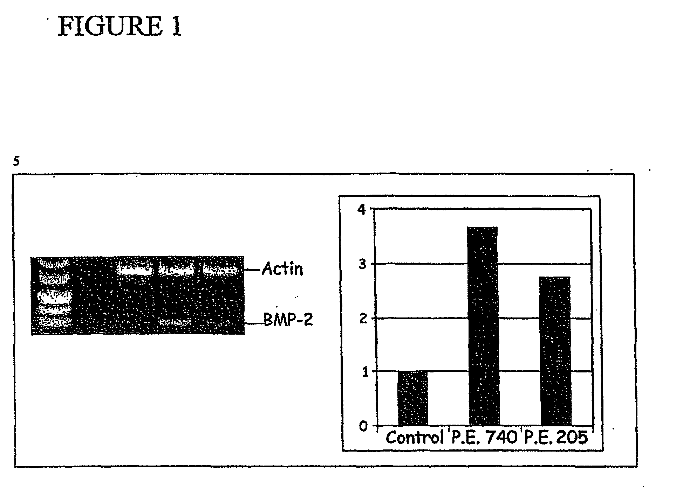

**Figure 1** **:** Measurement of endogenous BMP-2 mRNA expression in hPOB-tert cells by RT-PCR following treatment with extracts from *Lindera benzoin* (P.E. 740, 50 µg/ml) or *Cyperus Rotundus* (P.E. 205, 10 µg/ml) for 48h and showing stimulation of BMP-2 by 3.8 and 2.8-fold of control, respectively. The validation of this assay has been performed with lovastatin (0.5 µg/ml) as a positive control showing induction of BMP-2 by 2.5 fold.

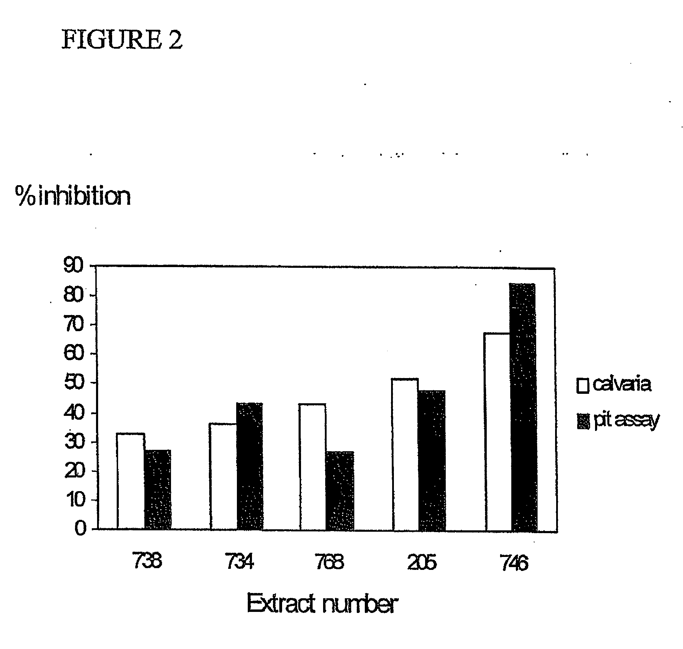

**Figure 2** **:** Comparison of measured inhibition values for the calvaria Assay (A) and the Pit Assay (B) for the extracts of *Ocimum gratissimum* (73 8), *Amelanchier alnifolia* (734), *Glycine max* (768), *Cyperus rotundus* (205), *Carthamus tinctorius* (746).

### Examples

The following examples illustrate the claimed invention, where the plant or plant extract is *Carum.* Other plants and plant extracts are exemplified for comparison.

### Example 1 : Assays on bone formation and bone resorption

### 1. bone formation

91 extracts were screened for bone formation through the BMP-2 (bone morphogenic protein) gene reporter assay and for bone resorption through the Calvaria assay.
These 91 extracts correspond to 30 different plants.

### Materials and methods

### • Preparation of extracts for screening assays:

The ground plant material is defatted with hexane then extracted with a mixture of alcohol and water, with different percentages of water from 10 to 90%, preferably with 50%. The alcohols can be methyl or ethyl alcohols, giving the extract 1a.

On an aliquot of the residue of this first extract, an enzymatic hydrolysis is carried out with α and β glucosidases. Enzymes can be replaced by acidic conditions. The operation may be done under mild conditions (room temperature) or through reflux with different acid concentrations. The aqueous hydrolysed phase is extracted with a non-miscible solvent, preferably ethylacetate to give the extract 2a.

The extract can be dried, freeze-dried or supplied as a liquid form.
In some cases, polyphenols can be discarded through a polyvinylpolypyrrolidone (PVPP) treatment, avoiding artefact with the screening assays.

Following the extract preparation, each extract was weighed, redissolved in dimethylsulphoxide (DMSO) to a final concentration of 20 mg/ml and stored in aliquots at -20°C. This was used as a stock solution and was subsequently diluted in media for each assay. A range of doses was tested in the assay systems.

### • Bone formation assay

BMP-2 luciferase assay - The activity of the extracts was determined using 2T3 cells containing the BMP-2 promoter operatively linked to the luciferase gene. An increase in luciferase activity in cell lysates reflects an increase in BMP-2 promoter activity. The extracts were assayed at an initial dilution of 100 µg/ml with ½ dilutions down to 0.2 µg/ml BMP-2 promoter activity was measured by measuring the luciferase activity in cell extracts.

13 plants gave a significant positive result in stimulation of BMP-2 expression (Table 1)

**Table 1**

| **Latin name** | **English name** | **part** | **conc µg/ml** | **Active extract/n°** |
|---|---|---|---|---|
| *Acorus calamus* | sweet flag | rhizome | 5 | MeOH/water/731 |
| *Amelanchier* | service | fruit | 10 | MeOH/water/219 |
| *ovalis* | berry | | | |
| *Artemisia* | mugwort/w | aerial parts | 10 | Ethylacetate/225 |
| *vulgaris* | ormwood | | | |
| *Cyperus rotundus* | nutgrass | rhizome | 10 | Ethylacetate/205 |
| *Taraxacum* | common | leaves | 50 | ethylacetate/750 |
| *officinalis* | dandelion | | | |
| *Lindera benzoin* | spice bush | aerial parts | 50 | ethylacetate/740 |
| *Prunus persica* | peach | seed | 25 | ethylacetate/772 |
| *Glycine max* | soybean | cell cultures | 50 | ethylacetate/768 |
| *Iris pallida* | sweet iris | tubers | 100 | MeOH/water/239 |
| *Rosmarinus* | rosemary | leaves | 50 | MeOH/water2004 |
| *officinalis* | | | | ethylacetate/2005 |
| *Carvi* | caraway | seeds | 25 | ethylacetate/2074 |
| *Thyme* | thyme | leaves | 25 | ethylacetate/2067 |
| *Mentha spicata* | mint | leaves | 100 | ethylacetate/2072 |
| *Vitis vinifera* | grape | fruit | 100 | ethylacetate/2069 |

Examples of new preparations of the same plants and subfractions thereof which stimulate BMP-2 are:
*Lindera benzoin,* active extract/n° ethylacetate/740/2059; Active subfraction/n° 2060 *Taraxacum officinalis,* active extract/n° ethylacetate/750/2034; Active subfraction/n° 2035
*Cyperus* rotundus, active extract/n° ethylacetate/205/2011; Active subfraction/n° 2012,2013
*Iris pallida,* active extract/n° MeOH/water/239; Active subfraction/n° 760/762/2021, 2022

Subfractions were prepared by fractionation on reverse phase silica gel cartridge with elution by solvents of varying polarity. The pure soy isoflavones genistein and daidzein (10⁻⁶M) stimulated BMP-2 but estradiol did not.

BMP-2 induction seems to be not restricted to estrogenic-like activity, as it is stimulated by phytoestrogen but not by estrogen itself It means that the activity of phytoestrogen (such as genistein and daidzein) may be mediated by a nonestrogenic mechanism. BMP-2 promoter activity is not stimulated by estradiol, thus estrogenicity of plant compounds is not required to be active in this test.

**Example of bone formation in calvaria organ culture**

| Plant | Active extract no | concentration µg/ml) |
|---|---|---|
| *Glycine max* | ethylacetate/2001 | 10, 50 |
| *Rosmarinus officinalis* | MeOH/water/2004 | 10, 50 |
| *Rosmarinus officinalis* | ethylacetate/2005 | 10 |
| *Cyperus rotundus* | subfraction/2012 | 10, 50 |
| *Iris pallida* | subfraction/2022 | 10 |
| *Thyme* | ethylacetate/2067 | 10 |
| *Carvi* | ethylacetate/2074 | 10 |

The method used is described in Science 286: 1946-1949 (1999). Extracts were assessed in a 4 day in vitro neonatal murine calvarial assay. Bones were incubated with the extracts for the entire 4 days. Bone formation was assessed by histology.

### 2. Bone resorption, calvaria assay

The ability of the extracts to inhibit IL-1 (10⁻¹⁰ M) stimulated bone resorption was assessed in the neonatal bone resorption assay. Each extract was assessed for its capacity to inhibit bone resorption at 10 µg/ml

The ability of the extracts prepared as in example 1, to inhibit IL-1 (10⁻¹⁰ M) stimulated bone resorption was assessed in the neonatal bone resorption assay. Each extract was assessed for its capacity to inhibit bone resorption at 10 µg/ml

The plant extracts found positive are listed in Table 2:

**Table 2.**

| **Latin name** | **English name** | **part** | **Active extract/n°** |
|---|---|---|---|
| *Amelanchier alnifolia* | serviceberry | fruit | ethylacetate/734 |
| *Ocimum gratissimum* | basil sp. | leaves | MeOH/H₂O/737 |
| *Ocimum gratissimum* | basil sp. | leaves | ethylacetate/738 |
| *Carthamus tinctorius* | safflower | seed | ethylacetate/746 |
| *Cyperus rotundus* | nutgrass | rhizome | ethylacetate/205 |
| *Glycine max* | soybean | cell cultures | 768 |

These plants were active in the bone resorption assay: *Amelanchier alnifolia, Ocimum gratissimum* and *Carthamus tinctorius and Glycine max. Cyperus rotundus* inhibited bone resorption and induced BMP-2.

### Example 2: Effect of plant extracts on endogenous BMP-2 expression in human osteoblast cells

The plants found positive for BMP-2 induction in example 1 were further tested in a human periosteal /preosteoblast cell line, hPOB-tert for their ability to induce the endogenous expression of BMP-2. This test in osteoblast cells confirmed the results shown in example 1.

For example, treatment of hPOB-tert cells with extracts of *Lindera benzoin* (extract 740, 50 µg/ml), and of *Cyperus rotundus* (extract 205, 10 µg/ml,) for 48h stimulated BMP-2 expression 3.8 and 2.8 fold of control (see Figure 1). The validation of this assay has been performed with lovastatin (0.5 µg/ml) as a positive control showing induction of BMP-2 by 2.5 fold.

At confluence, cells were incubated with 0.05 µg/ml Lovastatin or with the plant extracts. Total RNA was extracted with TRIzol Reagent (Gibco). 10 µg RNA were reverse transcribed using the 1st Strand cDNA Synthesis Kit (Boehringer). BMP-2 cDNA sequences were amplified for 35 cycles at an annealing temperature of 55°C using specific oligonucleotide primers (5':TTGCGGCTGCTCAGCATGTT; 3':CATCTTGCATCTGTTCTCGGAA). PCR products were separated by agarose gel electrophoresis and detected by ethidium bromide staining. Quantification was performed using NIH Image Software and normalizing results with Actin as housekeeping gene.

Results are shown in Figure 1.

### Bone resorption

The plant extracts found positive in the calvaria assay were retested in a second assay of bone resorption, namely in the pit assay using rabbit bone mixed cells cultured on bovine bone slices (Tezuka K., et al., 1992, Biochem Biophys. Res. Commun. 186(2):911-7 and Lorget F., et al., 2000, Biochem Biophys. Res. Commun. 268(3):899-903). Resorption pits were visualized by staining for TRAP (tartrate resistant acid phosphatase) . Positive cells and counted.

A comparison of activity of the extracts at 10 µg/ml in the two assay systems is shown in Figure 2.

### Example 3: Dry pet food

A feed mixture is made up of about 58% by weight of corn, about 5.5% by weight of corn gluten, about 22% by weight of chicken meal, 2.5% dried chicory, about 10% of cyperus rotondus tubers, salts, vitamins and minerals making up the remainder.

The feed mixture is fed into a preconditioner and moistened. The moistened feed is then fed into an extrader-cooker and gelatinised. The gelatinised matrix leaving the extruder is forced through a die and extruded. The extrudate is cut into pieces suitable for feeding to dogs, dried at about 110°C for about 20 minutes, and cooled to form pellets.

This dry dog food has a positive effect on bone and cartilage health and increase their mobility.

### Example 4: Wet canned pet food with supplement

A mixture is prepared from 73 % of poultry carcass, pig lungs and beef liver (ground), 16 % of wheat flour, 2 % of dyes, vitamins, and inorganic salts. This mixture is emulsified at 12°C and extruded in the form of a pudding, which is then cooked at a temperature of 90°C. It is cooled to 30°C and cut in chunks. 45 % of these chunks are mixed with 55 % of a sauce prepared from 98 % of water, 1 % of dye, and 1 % of guar gum. Tinplate cans are filled and sterilised at 125°C for 40 min.

As a supplement to be mixed with the pet-food before serving, additional packaging (e.g. sachet) contains 25 g of powdered *Cyperus Rotundus* aerial parts to be added to the daily food. The corresponding amount for the pet is about 25 g / day and this can be supplied as a supplement with (e.g. on top of) the can.

## Claims

1. A composition containing a plant or a plant extract containing phytochemicals having the ability to stimulate bone morphogenic protein, wherein the plant or plant extract is *Carum,* for use in;
helping bone regeneration during fracture healing;
helping to increase bone formation and bone mineral density during growth;
optimizing peak bone mass;
helping to build cartilage;
helping to prevent osteoarthritis which results in a better activity or mobility of the individual;
the treatment, alleviation and/or prophylaxis of osteoarthritis;
treating or preventing osteoporosis;
in humans or pets.

2. Use of a plant or a plant extract containing phytochemicals having the ability to stimulate bone morphogenic protein, wherein the plant or plant extract is *Carum,* for the manufacture of a composition for use in;
helping bone regeneration during fracture healing;
helping to increase bone formation and bone mineral density during growth;
optimizing peak bone mass;
helping to build cartilage;
helping to prevent osteoarthritis which results in a better activity or mobility of the individual;
the treatment, alleviation and/or prophylaxis of osteoarthritis;
treating or preventing osteoporosis;
in humans or pets.

3. A composition according to claim 1 or use according to claim 2, wherein the plant is from any part of the plant source, leaves, tubers, fruits, seeds, roots, grains, embryos or cell cultures.

4. A composition or use according to one of claims 1 to 3, wherein the plant or plant extract is aerial parts of *Carum carvi.*

5. A composition or use according to one of claims 1 to 4, wherein the phytochemical is carvone.

6. A composition or use according to one of claims 1 to 5, in which the plant or plant extract is used alone or in association with other plants such as chicory, tea, cocoa, or with other bioactive molecule such as antioxidants, fatty acids, prebiotic fibers, glucosamine, chondroitin sulphate for example.

7. A composition or use according to one of claims 1 to 6, wherein the composition is in the form of a nutritionally balanced food or pet food, a dietary supplement, a treat or a pharmaceutical composition.

## Patentansprüche

1. Zusammensetzung, die eine Pflanze oder einen Pflanzenextrakt enthält, die/der Phytochemikalien enthält, die in der Lage sind, Knochen-Morphogenese-Protein zu stimulieren, worin die Pflanze oder der Pflanzenextrakt *Carum* ist, zur Verwendung bei
der Förderung der Knochenregeneration während einer Frakturheilung;
der Förderung der Zunahme der Knochenbildung und Knochenmineraldichte während des Wachstums;
der Optimierung der maximalen Knochenmasse;
der Förderung des Aufbaus von Knorpel;
der Förderung der Prävention von Osteoarthritis, was zu einer besseren Aktivität oder Mobilität des Individuums führt;
der Behandlung, Linderung und/oder Prophylaxe von Osteoarthritis;
der Behandlung oder Prävention von Osteoporose;
bei Menschen oder Haustieren.

2. Verwendung einer Pflanze oder eines Pflanzenextrakts, die/der Phytochemikalien enthält, die in der Lage sind, Knochen-Morphogenese-Protein zu stimulieren, worin die Pflanze oder der Pflanzenextrakt Carum ist, zur Herstellung einer Zusammensetzung zur Verwendung bei
der Förderung der Knochenregeneration während einer Frakturheilung;
der Förderung der Zunahme der Knochenbildung und Knochenmineraldichte während des Wachstums;
der Optimierung der maximalen Knochenmasse;
der Förderung des Aufbaus von Knorpel;
der Förderung der Prävention von Osteoarthritis, was zu einer besseren Aktivität oder Mobilität des Individuums führt;
der Behandlung, Linderung und/oder Prophylaxe von Osteoarthritis;
der Behandlung oder Prävention von Osteoporose;
bei Menschen oder Haustieren.

3. Zusammensetzung nach Anspruch 1 oder Verwendung nach Anspruch 2, worin die Pflanze von einem jedweden Teil der pflanzlichen Quelle, Blätter, Knollen, Früchten, Samen, Wurzeln, Körnern, Keimlingen oder Zellkulturen, stammt.

4. Zusammensetzung oder Verwendung nach einem der Ansprüche 1 bis 3, worin die Pflanze oder der Pflanzenextrakt oberirdische Teile von *Carum carvi* darstellt.

5. Zusammensetzung oder Verwendung nach einem der Ansprüche 1 bis 4, worin die Phytochemikalie Carvon ist.

6. Zusammensetzung oder Verwendung nach einem der Ansprüche 1 bis 5, in der die Pflanze oder der Pflanzenextrakt allein oder zusammen mit anderen Pflanzen, wie zum Beispiel Zichorie, Tee, Kakao, oder mit einem anderen bioaktiven Molekül, wie zum Beispiel Antioxidantien, Fettsäuren, präbiotischen Ballaststoffen, Glucosamin, Chondroitinsulfat beispielsweise, verwendet wird.

7. Zusammensetzung oder Verwendung nach einem der Ansprüche 1 bis 6, worin die Zusammensetzung in Form eines ernährungsphysiologisch ausgewogenen Lebensmittels oder Haustierfutters, eines Nahrungsergänzungsmittels, eines Genussmittels oder einer pharmazeutischen Zusammensetzung vorliegt.

## Revendications

1. Composition contenant une plante ou un extrait végétal contenant des agents phytochimiques ayant la capacité de stimuler une protéine morphogénique osseuse, dans laquelle la plante ou l'extrait végétal est Carum, destinée à être utilisée dans :
l'aide à la régénération osseuse au cours de la guérison d'une fracture ;
l'aide à augmenter la formation osseuse et la densité minérale osseuse au cours de la croissance ;
l'optimisation de la masse osseuse maximale ;
l'aide à la construction de cartilage ;
l'aide à la prévention de l'ostéoarthrite, ce qui conduit à une meilleure activité ou mobilité de l'individu ;
le traitement, le soulagement et/ou la prophylaxie de l'ostéoarthrite ;
le traitement ou la prévention de l'ostéoporose ;
chez l'homme ou l'animal.

2. Utilisation d'une plante ou d'un extrait végétal contenant des agents phytochimiques ayant la capacité de stimuler une protéine morphogénique osseuse, dans laquelle la plante ou l'extrait végétal est Carum, pour la fabrication d'une composition destinée à être utilisée dans :
l'aide à la régénération osseuse au cours de la guérison d'une fracture ;
l'aide à augmenter la formation osseuse et la densité minérale osseuse au cours de la croissance ;
l'optimisation de la masse osseuse maximale ;
l'aide à la construction de cartilage ;
l'aide à la prévention de l'ostéoarthrite, ce qui conduit à une meilleure activité ou mobilité de l'individu ;
le traitement, le soulagement et/ou la prophylaxie de l'ostéoarthrite ;
le traitement ou la prévention de l'ostéoporose ;
chez l'homme ou l'animal.

3. Composition selon la revendication 1 ou utilisation selon la revendication 2, dans laquelle la plante provient de toute partie de la source végétale, des feuilles, des tubercules, des fruits, des graines, des racines, des grains, des embryons ou de cultures cellulaires.

4. Composition ou utilisation selon l'une des revendications 1 à 3, dans laquelle la plante ou l'extrait végétal est des parties aériennes de Carum carvi.

5. Composition ou utilisation selon l'une des revendications 1 à 4, dans laquelle l'agent phytochimique est la carvone.

6. Composition ou utilisation selon l'une des revendications 1 à 5, dans laquelle la plante ou l'extrait végétal est utilisé seul ou en association avec d'autres plantes telles que la chicorée, le thé, le cacao ou avec une autre molécule bioactive telle que des antioxydants, des acides gras, des fibres prébiotiques, de la glucosamine, du sulfate de chondroïtine par exemple.

7. Composition ou utilisation selon l'une des revendications 1 à 6, dans laquelle la composition est sous la forme d'un aliment ou aliment pour animaux équilibré du point de vue nutritionnel, d'un supplément diététique, d'une friandise ou d'une composition pharmaceutique.
